# EUROPÄISCHE PATENTSCHRIFT

(11) **EP 0 705 111 B1**
(45) Veröffentlichungstag und Bekanntmachung des Hinweises auf die Patenterteilung: **20.08.2008**
(21) Anmeldenummer: 94919641.4
(22) Anmeldetag: 19.06.1994
(51) Int. Cl.: A61K 45/06, A61K 31/495

(54) **MITTEL ZUR BEEINFLUSSUNG VON HYPERAKTIVIERTEN IMMUNOLOGISCHEN EFFEKTORZELLEN**
AGENT WITH INFLUENCES HYPERACTIVE IMMUNOLOGICAL EFFECTOR CELLS
AGENT PERMETTANT D'INFLUER SUR DES CELLULES EFFECTRICES IMMUNOLOGIQUES HYPERACTIVEES

(30) Priorität: 23.06.1993 DE 4320878; 25.07.1993 DE 4324877; 07.04.1994 DE 4411956
(43) Veröffentlichungstag der Anmeldung: 10.04.1996
(73) Patentinhaber: LESKOVAR, Peter, D-83026 Rosenheim (DE)
(72) Erfinder: LESKOVAR, Peter, D-83026 Rosenheim (DE)
(74) Vertreter: Leissler-Gerstl, Gabriele
(86) Internationale Anmeldenummer: PCT/EP1994/001992
(87) Internationale Veröffentlichungsnummer: WO 1995/000175

(56) Entgegenhaltungen:
- CEPHALALGIA (NORWAY), 1991, VOL. 11, SUPPL. 11, PAGE(S) 166-167, Viswanathan K.N. et al 'Cinnarizine -propranalol in migraine prophylaxis - A double blind clinical study'
- J. IMMUNOL. (USA), 1982, VOL. 129, NO. 1, PAGE(S) 287-296, Katz P. et al 'Mechanisms of human cell-mediated cytotoxicity. I. Modulation of natural killer cell activity by cyclic nucleotides'

## Beschreibung

Die Erfindung betrifft die Verwendung eines Mittels zur Beeinflussung von hyperaktivierten immunologischen Effektorzellen.

Bei verschiedenartigen Zuständen und Erkrankungen des Menschen sind die immunologischen Effektorzellen durch zuviele Reize, z.B. durch Cytokine, hyperaktiviert und können daher auf neue spezifische Reise nicht mehr reagieren. Das Immunsystem wird dadurch negativ beeinträchtigt oder sogar ausgeschaltet. Dieser Zustand kann hervorgerufen werden, wenn eine Infektion zu lange andauert durch persistierende Stimulation oder wenn Zellen kurzzeitig überaktiviert werden durch zu starke Ausschüttung von endogenen Cytokinen. Unter des Begriff "immunologische Effektorzellen" werden hier z.B. T-Zellen, Makrophagen/Monozyten, HK-Zellen und andere immunologisch wirksame Zellen verstanden.

An vielen immunologischen Prozessen ist auf der zellulären Ebene das aus Adenosintriphosphat (ATP), z.B. durch Einwirkung von Adenylatcyclase (AC), gebildete cyclische Adenosinmonophosphat (cAMP) beteiligt. cAMP nimmt als "second messenger" eine zentrale Rolle in der hormonalen Regulation und in Stoffvechsel ein durch Aktivierung von Proteinkinasen, z.B. Proteinkinase A (PKA). PKA phosphoryliert dann Proteine, die wiederum die Immunantwort supprimieren. Dadurch wirkt eine Hyperaktivierung der Effektorzellen für die Immunfunktionen suppressiv. Zur Steuerung dieser Reaktionskaskade gibt es eine antagonistische Bildung von cyclischem Guanosinmonophosphat (cGHP), das dem cAMP entgegenwirkt.

Diese Reaktionskaskade wird auch beeinflußt durch die Gruppe der G-Protein gekoppelten Rezeptoren, zu denen Rezeptoren, wie adrenerge, Muscarin-, Histamin-, Serotonin- and Adenosinreseptoren gehören, G-Proteine (Guanin-Nucleotide-bindende Proteine) können die Bildung eines second messenger stimulieren (Gₛ) oder hemmen (Gᵢ). Durch Beeinflussung von Gₛ- und Gᵢ-Rezeptoren kann daher auch die Bildung von AC und damit von cARP beeinflußt werden.

Zustände bei denen das natürliche Gleichgewicht nicht mehr vorliegt sind beispielsweise Krebs, Virenerkrankungen und Autoimmunerkrankungen, einschließlich der auslösenden und krankheitserhaltenden autoimmunen Komponente der Arteriosklerose.

Es war nun Aufgabe der Erfindung, ein Mittel bereitzustellen, un derartige Krankheitszustände zu bekämpfen und ein durch Hyperaktivierung supprimiertes Immunsystem wieder für Reize und eine normale Immunantwort ansprechbar zu machen.

Diese Aufgabe wird gelöst durch ein Mittel zur Beeinflussung von hyperaktivierten immunologischen Effektorzellen, das
I) einen calciumantagonisten und
(II) einer Antagonisten von β-adrenegen Receptore als ein den intrazellulären Quotienten von cAMP/cGHP verminderndes Mittel umfaßt.

Überraschenderweise wurde festgestellt, daß mit einer derartigen Kombination die Hyperaktivierung von Effektorzellen vermindert oder aufgehoben werden kann. Dadurch werden diese Zellen wieder bereit für die Aufnahme spezifischer Reize und können wieder eine normale Immunreaktion liefern. Es wurde gefunden, daß hyperaktivierte Zellen zuviel Calcium enthalten und einen zu hohen Quotienten von cAMP/cGMP aufweisen. In einer ersten Ausführungsform besteht daher das Prinzip des erfindungsgenäßen Mittels darin, den Calciumfluß in die Zelle zu verhindern und den pathologisch veränderten Quotienten von cAMP/cGMP beeinflußt wird. Dies wird erreicht durch die erfindungsgemäße Kombination der Komponenten (I) und (II).

Überraschenderweise wird es damit möglich, so unterschiedliche Krankheiten, wie Tumoren, autoimmune Erkrankungen, Arteriosklerose, die möglicherweise zu ihrer Auslösung einen autoimmunen Promotor benötigt, ferner bakterielle, virale einschließlich retrovirale Infektionen und einige auf immunologischen Entgleisungen bzw. Deviationen beruhende, "moderne" Krankheiten, z.B. das CFS (chronic fatigue syndrome) zu behandeln. All diese Zustände und Erkrankungen könnten - immunologisch gesehen - gemeinsame Dysregulationselements aufweisen. Diese gemeinsamen Elemente sind z.B. eine persistierende oder überschießende Aktivierung gewisse Leukozyten-Subpopulationen, weist Makrophagen und Helfer-T-Zellen, nicht selten auch Suppressorzellen. Bei einer simultanen Hyperaktivierung von Makrophagen und Helfer-T-Zellen, wie sie auch bei HIV-Patienten beobachtet wurde, kann es zur gegenseitigen Stimulierung beider voneinander abhängiger Leukozyten-Subpopulationen kommen.

Ein weiteres gemeinsames Element ist die Deblockierung von blastogen prätransformierten T4- und Plasma-(B)-Zellen als Folge vorausgegangener, länger andauernder latenter oder manifester Immunsuppression; sie basiert auf der "Ausdünnung" der autoantigen- bzw. patbogenspezifischen Überwacherzellen oder Suppressor-T-Zellen.

Von molekular-biologischen Gesichtspunkt her ist den unterschiedlichsten, oben erwähnten Krankheiten die Persistens eines erhöhten intrazellulären ca²⁺ᵢ-Spiegels gemeinsam, die einerseits zur immunsuppressiven Gegenregulation und andererseits zur herabgesetzten Immunstimulierbarkeit gegenüber neuen antigenen Reizen führt. Dies resultiert nicht nur in einer Beeinträchtigung des bestehenden Sets an immunkompetenten Zellen, sondern zusätzlich in einer gestörten Nachrekrutierung neuer, noch steuerbarer Immunozyten.

Die erfindungsgemäße Kombination der Komponenten I und II sorgt für eine Deblockierung der falsch programmierten Immunozyten oder Effektorzellen, vor allem der Zellen in hyperaktivierten Zustand, und bat daher Einfluß auf die "wound healing"- bzw. "tissue repair"-Funktion von umprogrammierten Makrophagen und Cytokine-hypersezernierenden Helfer-T-Zellen.

Diese Beruhigung der hyperaktivierten oder persistierend aktivierten Effektorzellen wird einerseits durch Komponente I erreicht durch Verhinderung der Ca²⁺ᵢ-Starre, d.h. einer Blockierung der Zellüberladung mit Ca²⁺ᵢ-Ionen; andererseits wird die Ca²⁺ᵢ-Abhängigkeit durch Regulierung des intrazellulären pHᵢ-Wertes reguliert. Ein weiterer Mechanismus ist die Kontrolle des Elektrolyttransportes durch die Zellmembran, d.h. eine Beeinflussung der Ca²⁺-, Na⁺- und K⁺-Kanäle bzw. der Austauschmechanismen für Na⁺/K⁺, Na⁺/H⁺, K⁺/H⁺, HCO₃⁻/Cl⁻, Ca²⁺/Na⁺ oder Ca²⁺/H⁺ bzw. verschiedener ATPasen, Anti- und Symport.

Mit der erfindungsgemäßen Kombination konnten bei Tumorpatienten Verkleinerungen von Tumor oder Metastasen un ein bis zwei Drittel innerhalb von einigen Wochen erzielt werden, ohne gleichzeitige Radio- oder Chemotherapie. Auch bei Patienten mit Autoimmun-Erkrankungen, z.B. multipler Sklerose oder CFS wurden überraschende therapeutische Erfolge erzielt.

Die in folgenden angegebenen einzelnen Beispiele für die Komponenten I und II können jeweils untereinander kombiniert werden. Es ist auch möglich ihre Wirkung in einer weiteren Ausführungsform der Erfindung zu erweitern durch Kombination mit bekannten Immunstimulatoren, Immunodulatoren (BRMS) bei neoplastischen und autoimmunen Erkrankungen, ferner bei arteriosklerotischen Gefäßveränderungen und ebenso bei mit Makrophagen- und T-Zell-Hyperaktivierung zusammenhängenden, auf Plasma-Destabilisierung beruhenden Erkrankungen, wie Amyloidose, Alzheimer Krankheit, und bei CFS. Auch bei bakteriellen und viralen Infektionen sind diese Kombinationen therapeutisch nützlich. Hierdurch kann die initiale immunologische Entgleisung, d.h. die Hyperaktivierung von Leukozyten-Subpopulationen rückgängig gemacht werden; die Restaurierung des ursprünglichen Immunstatus kann dann spontan erfolgen.

Das Therapieschema kann beispielsweise 2- oder 3-stufig sein. Dabei kann z.B. in einer ersten Phase eine Deblockierung von immunkompetenten, aber falsch programmierten Effektorzellen, primär Makrophagen und T-Zellen, und in einer zweiten Phase eine kontrollierte Stimulierung der Immunozyten erfolgen. In einer weiteren Ausführungsform kann sich dann noch als dritte Phase ein Einfrieren des kontrolliert stimulierten Zellzustandes, praktisch eine Verlängerung der zweiten Phase, anschließen. Für die erste Phase kann dazu das erfindungsgemäße Mittel eingesetzt werden. Für die zweite Phase sind bekannte Immunstimulantien geeignet und für die dritte Phase kann wieder das erfindungsgemäße Mittel eingesetzt werden, jedoch in geringerer Dosierung, z.B. 20 bis 50t der in der ersten Phase verwendeten Menge.

Einzelheiten über die Deblockierung der immunkompetenten Zellen in hypoxischen und/oder azidifirierten Milieu von chronisch entzündeten Geweben, incl. nekrotisierendes Tumorgewebe werden beschrieben. Hyper- oder persistierend aktivierte Makrophagen verbrauchen 10- bis 20mal mehr Sauerstoff als ruhende, in Anwesenheit von gamma-Interferon steigt dieser O₂-Verbrauch noch wesentlich stärker an.

Bei der Phagozytose von opsonisierten Partikeln (Bakterien, Latex usw.) sowie von Immunkomplexen via den Fc-gamma-Rezeptor schaltet der Makrophage auf den Embden-Mayerhof-glykolytischen Glucoseabbau um. Hierdurch wird der molekulare Sauerstoff für die Bildung von mikrobiziden Sauerstoff-Radikalen (ROI) zur Verfügung gestellt. Auch die Bildung von Leukotrien C, D und E, die ein reduziertes Glutathion voraussetzt, wird der Übertragung von Elektronen bzw. Protonen an Sauerstoff-Radikale (mittels Cytochrom P450) geopfert. Das statt Leukotrien C, D und E gebildete Leukotrien B stimuliert direkt die Cytochrom P450 vermittelte ROI-Bildung.

Diese durch Opsonisierung anfoktroierte Umschaltung zur Glykolyse führt via Milchsäure-Überproduktion und -Sekretion zur starken Ansäuerung des Mikromilieus um hyperaktivierte Makrophagen. Zusammen mit der hypoxischen Gewebeansäuerung führt dies zum lokalen pH-Abfall und hiermit zur Hemmung der Immunozyten-Funktion. Die gestörte Bildung von Leukotrien C, D und E, die durch ROI-Produkte noch zusätzlich beeinträchtigt wird, resultiert in gehemmter Glykosylierung und Sezernierung von cytokinen und Immunglobulinen.

Die durch gestörte Angiogenese und hyperaktivierte Nekrophagen entstandene Hypoxie in Entzündungsgewebe, z.B. nekrotisierendem Tumorgewebe führt über mehrere Schritte zum Abfall des intrazellulären pH-Wertes. Dieser äußert sich zuerst in einer starken Hemmung der Zellaktivität (unterhalb von pH 6,8) und endet bei weiteren Abfall in Zelltod.

Ohne an eine Theorie gebunden zu sein, wird angenommen, daß sowohl der apoptotische als auch der autolytische Zelltod auf einen Abfall des intrazellulären pHs basiert, wodurch lysosomale Enzyme aktiv werden. Zuvor versagt die H⁺-Pumpe, die in den Lysosomen einen pH von 4,5 bis 5,0 aufrechterhält.

Von den Myokardzellen her weiß man, daß der Zelltod in Anwesenheit des Sauerstoffs als terminalen Elektronen-Akzeptor der Atmungskettenphosphorylierung bevorzugt, bei gleichzeitiger Zellaktivierung bzw. gleichzeitig erhöhtem intrazellulärem Caᵢ-Spiegel eintritt. Darum sollte der, nur bei O₂-Anwesenbeit sinnvolle aerobe Glucoseabbauweg über den TCC/Citrat-Cyclus im hypoxischen bzw. ischämischen Gewebe medikamentös gehemmt werden. Hierdurch wird auch die Überproduktion von NADH₂ (und NADPH₂), die zur beschleunigten glykolytischen Milchsäureproduktion und hiermit pH-Abnahme führt, verhindert.

Es Können BRMs (Immunmodulatoren) mit deblockierenden Substanzen kombiniert bzw. die Deblockierung der immunkomponenten Zellen vor weiteren (immun)therapeutischen Schritten vorgenommen werden.

Einer der Hauptgründe der blockierten Funktion von immunkompetenten Zellen (mononukleären und polymorphkernigen Phagozyten, NK-Zellen, K-Zellen, T-Zellen) in situ/in vitro z.B. in Tumorläsionen, in chronisch-entzündlichen Geweben ist der niedrige cGHP-Spiegel bzw. der erhöhte cAMP-Spiegel im Cytosol der immunkompetenten Zellen.

Der erhöhte cAMP-Spiegel geht in erster Linie auf die Aktivierung der Adenylatcyclase durch hyperproduzierte Prostaglandine (PGE 2/E 1) zurück; diese Prostaglandine werden in erster Linie durch hyperaktivierte Makrophagen, Fibroblasten und Synovialzellen sezerniert und führen zusammen mit Catecholaminen via Aktivierung des beta-adrenergen Rezeptors zur erwähnten Adenylatcyclase-Aktivierung und hiermit zur cAHP-Bildung im Cytosol.

Der intrazelluläre cAMP-Anstieg hemnt verschiedene Zellfunktionen, u.a. die immunrelevanten Funktionen der mieten Immunozyten-Subklassen. Deshalb wird erfindungsgemäß die Induktion der Guanylcyclase und hiermit der Anstieg des intrazellulären cGMP-Spiegels in den immunkompetenten Zellen in vitro und in vivo bevorzugt angewendet.

Der besondere Vorteil dieser Aktivierungsart von Immunozyten ist in der Tatsache begründet, daß hierdurch in vitro und in vivo blockierte (präinaktivierte) Effektorzellen deblockiert bzw. von der Suppressor- zu der Effektor-Funktion umprogrammiert werden.

Für das erfindungsgemäße Mittel in Kombination geeignet sind die folgenden Substanzen die eingesetzt.

### (II) B-Blocker generell

Das Wirkungsprinzip ist hier die Verhinderung des intrazellulären Anstiegs von CAMP und der hiermit assoziierten Aktivität der A-Kinase (PKA).

### (III) Ca-Antagonisten generell

Das Wirkungsprinzip ist die Hemmung des Ca-Einstroms in die hyperaktivierte (hypoxische) Zelle, wodurch das Caᵢ-stimulierte Weiterlaufen des Krebscyclus bei mangelnder Präsenz des O₂-Elektronenakzeptors und mittelbar der pH-Abfall bzw. der Zelltod verhindert wird. In Falle der hyperaktivierten Makrophagen kann hierdurch die ROI-Produktion gehemmt werden. Diese ROI hemmen ihrerseits die Glycosylierung und hiermit die Sekretion von Cytokinen.

Für diese spezielle Kombination ist es bevorzugt, in zwei Stufen vorzugehen: Zuerst soll mit den besprochenen Ca-Antagonisten die Aktivität der "falsch programmierten", d.h. hyperaktivierten Makrophagen zum Stillstand gebracht werden.

Besonders bevorzugt wird für das erfindungsgemäße Mittel als Komponente I ein Calcium-Überladungsblocker, insbesondere Cinnarizin, und als Komponente II ein Antagonist von β-adrenergen Rezeptoren, insbesondere Propranolol, verwendet.

Anschließend werden einige bevorzugte Präparate genannt, die als Zweier-, Dreier- oder Mehrfachkombinationen (A+B, A+C..., B+C, B+D..., A-B-C, A-B-D...) für das erfindungsgemäße Mittel in betracht kon-nen. Ihr besonderer Vorteil liegt darin, daß sie als Einzelpräparate bekannt sind und direkt klinisch eingesetzt werden können. Sie können - als Kombinationspräparate - mit Immunmodulatoren (BRM) kombiniert werden.
1. Ca-Antagonisten (Zielsetzung: Verhinderung der intrazellulären Ca-Überladung und der hiermit assoziierten Hemmung von Guanylcyclase sowie der 5-Lipoxygenase einerseits und der Ca, genauer gesagt CaM (Ca-Modulin) stimulierten Ca-ATPase bzw. Adenylcyclase andererseits)
   1.1 auf der Nifedipin-Basis: Adalat (26.071) oder Aprical 5/-10/-retard (26.072), oder Bayotensin/mite (26.075)
   1.2 auf der Verapamil-Basis: Azupamil 40/ -80/ -120 (26.073) oder Dignover 40/ -80(2.081) oder Drostreakard 40/ -80/ -120 (26.083) oder Verapamil-ratiopharm (26.108)
   1.3 auf der Cinnarizin-Basis: Cinnarizin-ratiopharm (36.035) oder Cinnarizin Siegfried (36.036 oder Cinnarizin R.A.N. (36.034) oder Cinnacet (36.033) oder Cerepar (36.032).
2. beta-Blocker (Zielsetzung: Hemmung der cAMP-erhöhenden Sub-Rezeptoren für Katecholamin, PGE1/PGE2 sowie Histamin (H2-R)
   2.1 auf der Acetutolol-Basis: Meptal 400 (26.036) oder Prent 400 (26.040)
   2.2 auf der Metoprolol-Basis: Lopresor/-mite (26.035)
   2.3 auf der Propanolol-Basis: Indobloc 10/40/80 (26.032) oder Efektolol 10/40/80 (26.024) oder Elbrol 40/Elbrol 80 (26.027)
3. Kombinationspräparate: Ca-Antagonisten plus beta-Blocker
   Belnif (26.070) oder Tredalat (16.132) Nif-Ten 50 (16.131)

Von besonderen Interesse ist die erfindungsgemäße Kombination von β-Blocker und Ca-Kanalblocker (Ca-Antagonisten, wie Hifedipin, Verapamil und Diltiazem.

Zur Immuntherapie von neoplastischen und autoimmunen Erkrankungen sowie von bakteriellen und (retroviralen) Infekten durch Deblockierung des hyperaktivierten Zustandes von immunkompetenten Zellen wird das gleiche Prinzip verwendet, wie für die Verbesserung von konventionellen Vakzinen sowie für die Verhinderung der Abstoßungsreaktion bei Organtransplantationen.

Deblockierung von hyper- oder persistierend aktivierten immunkompetenten Zellen. Das eigentliche, bisher kaum beachtete Problem bei zahlreichen Erkrankungen wie Tumor, autoimmune Krankheiten, bakteriellen bzw. (retro)viralen Infekten (incl.HIV-Infekte) sowie bei CFS ("chronic fatigue syndrome") ist der hyper-, nicht hypoaktivierte Zustand von immunkompetenten Zellen. Durch persistierende Aktivierung einiger Subklone wird eine lokale oder systemische Suppression anderer Subklone und hiermit die fehlende Aktivierbarkeit (Hypoergie oder Anergie) für die krankheitsrelevanten Antigene (Pathogene) verursacht. Den oben erwähnten, auf ersten Blick grundunterschiedlichen Erkrankungen ist ein Element gemeinsam, nämlich eine vorausgehende, die Erkrankung mitinduzierende Phase der protrahierten Immunsuppression, die im Falle der Tumorerkrankung die immunologische Überwachung (immune surveillance) der (spontan) transformierten Körperzellen beeinträchtigt und bei Autoimmunkrankheiten die Deblockierung und klonale Postexpansion von autoaggressiven T- und B-Subklonen zur Folge hat.

Um die immunkompetenten Zellen wieder für krankheitsrelevante (Neo)antigensignale suszeptibel zu machen, müssen diese Zellen zuerst deblockiert oder aber inaktiviert bzw. eliminiert werden.

Diese Deblockierung gelingt erfindungsgemäß durch die Kombination der die "voltage-operated" und/oder "receptor-operated" Ca2+-Kanäle blockierenden Stoffe und Stoffgemische als Komponente I mit den das intrazelluläre cAMP-berabsetzenden bzw. den intrazellulären cAMP/cGMP-Quotienten erniedrigenden Stoffen und Stoffgemischen als Komponente II.

Die erfindungsgemäße Komponente I zur Blockierung von Ca2+-Kanälen kann "klassische" Ca2+-Antagonisten (Ca2+-Kanalblocker) aller Subtypen, z.B. Phenylalkylamine, Dihydropyridine, Benzothiazepine, Piperazine, Quinoxaline, Quinazoline, wie Bepridil und Perhexilin, umfassen. Zusätzlich kann gleichzeitig eine partielle Hemmung (Blockierung) von alpha- plus beta-Adrenozeptoren und/oder von H2- plus H1-Histamin-Rezeptoren und/oder A2- plus A1-Adenosin-Rezeptoren (mit entsprechenden niedrig-dosierten Antagonisten) und/oder 5HT(Serotonin)- Rezeptoren und/oder Rezeptoren verschiedener Entzündungsmediatoren (z.B.Bradykinin, Kinin-Kaskade, Komplement-Kaskade, speziell C5a, C4a,C3a, PAF etc.) erfolgen. Beispiele für bevorzugte Kombinationen sind weiter unten angeführt. Diese bevorzugten Stoffe und Stoffgemische können mit subdosierten Nitroverbindungen, z.B. Molsidomin sowie mit BRM (Immunmodulatoren) und/oder Cytokinen kombiniert werden.

Als Komponente II wird erfindungsgemäß ein β-Rezeptor-blocker als ein Mittel zur Reduktion des cAMP/cGMP-Quotienten verwendet. Verstärkt wird dieser Effekt, speziell die cAMP-Abnahme und die cGMP-Zunahme, d.h. Abfall des cAMP/cGMP-Quotienten durch gleichzeitige Gabe von Agonisten aller Gp-, Gi- oder Go-Protein-gekoppelten Rezeptoren und/oder von cGMP-erhöhenden, Cai-reduzierenden Nitroverbindungen (z.B.: Isosorbid-Mono- und Dinitrat, Glycerol-Trinitrat/Nitroglycerin, Erythrit-Tetranitrat, Pentaerythrit-Tetranitrat, Amylnitrit, Molsidomin).

Geeignet sind die Antagonisten Gs-gekoppelter Zellrezeptoren, deren normale, physiologische (endogene) Agonisten (Liganden) gleichzeitig an Gp-, Gi- oder Go-gekoppelte Zellrezeptoren binden; durch Verdrängung dieser endogenen Liganden von deren Gs-gekoppelten Zellrezeptoren interagieren sie verstärkt mit dem korrespondierenden Gp- bzw. Gi- bzw. Go-gekoppelten Rezeptor und erzielen gleichzeitig 2 Effekte, die cAMP-Abnahme und die cGMP-Zunahme im Cytosol der immunkompetenten Zielzelle. Beispiele solcher endogenen Agonisten sind Katecholamine (Adrenalin/ Epinephrin und Noradrenalin/ Norepinephrin), Histamin und Adenosin. So leiten die erf-gemäßen Antagonisten des β-Adrenozeptors (auf Immunozyten, z.B.T-Zellen und Monozyten/ Makrophagen) die Katecholamine, primär Adrenalin, vom β- zum alpha-Adrenozeptor um. Ähnlich wird durch Antagonisten des H2-Histamin-Rezeptors der endogene Ligand Histamin vom cAMP-erhöhenden H2-Rezeptor zum cAMP-herabsetzenden bzw. cGMP-erhöhenden H1-Rezeptor umgeleitet. Im Falle des endogenen Agonisten Adenosin bewirkt die Blockierung des A2-Subtyps des P1-Adenosin-Rezeptors (durch A2-Rezeptor-Antagonisten) die verstärkte Interaktion von Adenosin mit dem cAMP-unterdrückenden A1-Rezeptor.

Bevorzugt ist auch die Anwendung von Stoffen und Stoffgemischen, die neben den Ca-Kanälen auch Na-Kanäle reversibel hemmen können; das Wirkungsprinzip ist die Erhöhung des Ruhepotentials von immunkompetenten Zellen, welches bei hyperaktiviertem Zustand derselben herabgesetzt ist. Zu diesen Stoffen gehören einige Ca-Antagonisten, wie Cinnarizin, Flunarizin, Fendilin, Bepridil, Tiapamil und z.T. Verapamil und Gallopamil. Unter den Adrenozeptor-Blocker sind Sotalol und Propranolol von besonderem Interesse. Besonders bevorzugt ist die Kombination von Cinnarizin und Propranolol.

Der Kern der Erfindung ist die Erkenntnis, daß die Herabsetzung des intrazellulären cAMP-Spiegels bzw. des cAMP/cGMP-Quotienten mit der Blockierung der "voltage-operated" und/oder "receptor-operated" Ca2+-Kanäle zu kombinieren ist.

Wichtig ist auch die Notwendigkeit der allgemeinen Herabsetzung von unspezifischen und spezifischen Signalen, wodurch die Anergie von hyper- bzw. falsch aktivierten Effektorzellen (bei den oben aufgezählten Erkrankungen) durchbrochen und diese Effektorzellen (T-Zellen, Makrophagen/ Monozyten, NK-Zellen) für neue, krankheitsbezogene Signale wieder zugänglich werden. Diese Herabsetzung von unspezifischen ("unproduktiven" bzw. "Hintergrund"-Signalen) wird als "Herausfiltern" ("filtering out"/ "cutting off") von Signalen bezeichnet, erzielbar, wie erwähnt, durch sub-dosierte Antagonisten von z.B. β- plus alpha-Adrenozeptoren.

Herabsetzung der Hintergrund-Signale, d.h. "Herausfiltern" von unspezifischen (unproduktiven) transmembranalen Signalen, um die Suszeptibilität der immunkompetenten Zellen für spezifische, immunrelevante Signale zu erhöhen bzw.zu ermöglichen
1. Kombination von subdosierten β- (β1- plus β2-) und subdosierten alpha- (alpba1- plus alpha2-) Adrenozeptor-Blockern (Antagonisten) (Zielsetzung: Verhinderung des hypo- bzw. des anergen Zustandes von hyperaktivierten immunkompetenten Zellen durch Herabsetzung des Pegels von nichtspezifischen Hintergrundsignalen)
   1.1. Kombination von Präparaten auf der Pindolol-Basis (z.B. Durapindol / -15/-retard (26.039), oder Pinbetol/forte (26.067) ) plus Präparaten auf der Phenoxybenzamin-Basis (z.B. Dibenzyran 1/5 /10 (81.091)). Pindolol ist ein β1- plus β2-Sympatholytikum, Phenoxybenzamin dagegen ein alpha 1- plus alpha 2-Blocker. Empfohlene Dosierung: 1x 15 mg/d oder 3 x 5mg/ d Durapindol plus 1 x 5mg/d bzw. 2 - 3 x 1mg/d Dibenzyran
2. Kombination von subdosierten β- (β1- plus β2-) Adrenozeptor-Blockern mit subdosierten alpha1-Rezeptor-Antagonisten, die zusätzlich den H1-Histamin- und den 5HT (Serotonin)-Rezeptor blockieren (Zielsetzung: wie unter Punkt 1)
   2.1. Kombination von Präparaten auf der Pindolol-Basis (z.B.Durapindol/-15/-retard (26.039), oder Pinbetol/forte (26.067) ) plus Präparaten auf der Indoramin . HCl- Basis (z.B. Wydora/50 (16.039) ). Indoramin. HCl ist ein Antagonist des alpha1-Adrenozeptors, des H1-Histaminrezeptors sowie des 5HT (Serotonin)- Rezeptors. Empfohlene Dosierung: Durapindol (s.o.); Indoramin 1 x 25mg/d
3. Kombination von subdosierten β- (β1- plus β2-) Adrenozeptor-Antagonisten mit subdosierten alpha -(alpha1- oder alpha2-)Rezeptor-Blockern (Zielsetzung: wie unter Punkt 1 und 2)
   3.1. Kombination von Präparaten auf der Basis von 3.1.1. Alprenolol . HCl (z.B. Aptin (26.002)), 3.1.2. Bupranolol . HCl (z.B.Betadrenol 50/-100 (26.017)), 3.1.3. Penbutololsulfat (z.B. Betapressin (26.019)), 3.1.4. Bisoprololfumarat (z.B.Concor5/10 (26.025)) oder 3.1.5. Carteolol . HCl (z.B. Endak5/ 10 (26.046)) plus Präparaten auf der Basis von 3.1.1. Urapidil (z.B. Ebrantil 30/60/90 (16.032)), 3.1.2. Doxazosinmesilat (z.B. Cardular 1mg/-2mg/-4mg (16.029) oder Diblocin 1mg/-2mg/-4mg (16.030)) oder 3.1.3. Terazosin (z.B. Heitrin 1/2/5 (16.035)). Empfohlene Dosierungen: Aptin- Duriles 1 x 200mg/d; Betadrenol 1-2 x 50 mg/d; Betapressin 0,5-1 x 40mg/d; Concor 1 x 5 mg oder 1 x 10mg/d; Endak 5mg/d; Ebrantil 30mg/d; Cardular 1mg/d; Diblocin 1mg/d; Heitrin 0,5 - 1mg/d;
4. Kombination von subdosierten H2-und H1-Histaminrezeptor-Antagonisten (Zielsetzung : wie oben)
   4.1. Kombination von Präparaten auf der Basis von Cymetidin (z.B. Sigacimet 200/-400/-800 (59.102) oder Tagamet 200/-400/-800 (59.105) oder H2-Blocker- ratiopharm 200/400/800/1000 (59.096) plus Präparaten auf der Basis von 4.1.1. Oxatomid . H2O (z.B. Barpet (07004) ), 4.1.2. Brompheniramin-Hydrogenmaleat (z.B.Dimegan(07.005)), 4.1.3. Dimetindenmaleat (z.B. Fenistil (07.006)) oder 4.1.4. Terfenadin (z.B.Hisfedin (07.009)) Empfohlene Dosierungen: Sigacimet 1 x 200mg/d; Tagamet 1 x 200mg/d; H2-Blockerratiopharm 1 x 200mg/d; Barpet 1 x 30mg/d; Dimegan 1 x 12mg/d; Fenistil 1 x 1mg/d; Hisfedin 0,5- 1 x 60mg/d
5. Kombination von subdosierten Antagonisten des A2- plus A1- P1- purinergen (Adenosin) Rezeptors (Zielsetzung: wie oben)
   5.1. Kombination von Präparaten auf der Basis von Methylxanthinen (Theophyllin) (z.B. Aerobin mite (27.102) oder Contiphyllin Retardtabletten (27.113) oder Euphyllin N Tabletten (27.125) ) plus Präparaten auf der Basis von Ipratropiumbromid (z.B. Atrovent Kapseln (27.048) oder Itrop Filmtabletten (09028)). Empfohlene Dosierung: Aerobin 0,5-1 x 200mg/d; Contiphyllin Retardtabletten 0,5-1 x 300mg/d; Euphyllin N Tabletten 1 x 73mg/d; Atrovent Kapseln 0,5-1 x 200ug/d; Itrop Filmtabletten 0,5-1 x 10mg/d. Von Interesse sind auch Kombinationen mit Präparaten auf der Basis von Cromoglicinsäuredinatriumsalz und Ketotifenhydrofumarat.

Subdosierte Antagonisten unter Punkt 1, 2, 3 und 4 können kombiniert werden. In solchem Falle ist die Dosierung auf 5-50% der unter Punkt 1,2,3 und 4 aufgeführten Dosis zu reduzieren. Die stark subdosierten Antagonisten unter Punkt 1, 2, 3 und 4 können mit konventionellen Ca-Antagonisten und/oder mit subdosierten Agonisten von Gp- und Gi-gekoppelten Rezeptoren kombiniert werden.

Weitere Verbesserungen sowohl bei der neuartigen Therapie der oben genannten Erkrankungen als auch bei der Effizienz der konventionellen Vakzinen sind erfindungsgemäß durch Methylxanthine, intrazelluläres pH (pHi) korrigierende (erhöhende) Substanzen, Redoxpotential bzw. GSH/ GSSG bzw. NAD(P)H / NAD(P)+-Quotient verbessernde sowie Ionenhomöostase-, vor allem K+-Bilanz korrigierende Präparate zu erzielen.

Weil der hyperaktivierte Zustand von Effektorzellen (T-Zellen, Monozyten/Makrophagen, etc.) ein wichtiges Element in der Pathogenese (a) des Tumors (b) der Autoimmunkrankheiten (c) der arteriosklerotischen Gefäßveränderungen (d) der Infektionskrankheiten (inkl. HIV-Infektionen) ist, gelten die unter "Deblockierung von hyper- oder persistierend aktivierten immunkompetenten Zellen" beschriebenen Verfahren erfindungsgemäß für alle diese Erkrankungen.

## Patentansprüche

1. Verwendung einer Kombination aus
(I) einem Calcium-Antagonisten
(II) einem Antagonisten von β-adrenergen Rezeptoren als den intrazellulären Quotienten von cAMP/cGMP reduzierendem Mittel
zur Herstellung eines Arzneimittels zur Bekämpfung von Krebs, viralen und bakteriellen Erkrankungen und Autoimmnun-Erkrankungen.

2. Verwendung nach Anspruch 1, **dadurch gekennzeichnet, dass** der Calcium-Antagonist ein Calcium-Überladungsblocker ist.

3. Verwendung nach Anspruch 1 oder Anspruch 2, **dadurch gekennzeichnet, dass** der Calcium-Antagonist Cinnarizin ist.

4. Verwendung nach Anspruch 1, **dadurch gekennzeichnet, dass** der Antagonist für β-adrenerge Rezeptoren Propanolol ist.

## Claims

1. Use of a combination of
(I) a calcium antagonist and
(II) an antagonist of β-adrenergic receptors as agent to reduce the intracellular cAMP/cGMP ratio
for preparing a medicament for the treatment of cancer, viral and bacterial diseases and autoimmune diseases.

2. Use of claim 1, **characterized in that the** calcium antagonist is a calcium overload blocker.

3. Use of claim 1 or claim 2, **characterized in that** the calcium antagonist is cinnarizine.

4. Use of claim 1, **characterized in that** the antagonist of β-adrenergic receptors is propanolol.

## Revendications

1. Utilisation d'une combinaison d'un
(I) antagoniste du calcium
(II) antagoniste de récepteurs β-adrénergiques en tant que moyen de réduction du quotient intracellulaire de cAMP/cGMP
pour la fabrication d'un médicament pour combattre le cancer, les maladies virales et infectieuses et les maladies auto-immunes.

2. Utilisation selon la revendication 1, **caractérisée en ce que** l'antagoniste du calcium est un bloqueur de surcharge du calcium.

3. Utilisation selon la revendication 1 ou 2, **caractérisée en ce que** l'antagoniste du calcium est la cinnaricine.

4. Utilisation selon la revendication 1, **caractérisée en ce que** l'antagoniste pour les récepteurs adrénergiques est le propanolol.
